# EUROPEAN PATENT SPECIFICATION

(11) **EP 1 651 184 B1**
(45) Date of publication and mention of the grant of the patent: **22.05.2013**
(21) Application number: 04727072.3
(22) Date of filing: 13.04.2004
(51) Int. Cl.: A61K 9/70, A61K 9/00, A61K 31/485

(54) **FAST DISSOLVING ORALLY CONSUMABLE FILMS CONTAINING PHARMACEUTICALLY ACTIVE AGENTS**
SCHNELLZERFALLENDE ESSBARE FILME ENTHALTEND PHARMAZEUTISCHE WIRKSTOFFE
FILMS CONSOMMABLES ORALEMENT SE DISSOLVANT RAPIDEMENT ET CONTENANT DES AGENTS PHARMACEUTIQUEMENT ACTIFS

(30) Priority: 25.04.2003 US 423735
(43) Date of publication of application: 03.05.2006
(73) Proprietor: McNeil-PPC, Inc., New Brunswick NJ 08933 (US)
(72) Inventor: KULKARNI, Neema Mahesh, Randolph New Jersey 07869 (US); KUMAR, Lori Dee, Skillman New Jersey 08558 (US); SORG, Albert Frank, Columbia New Jersey 07832 (US)
(74) Representative: Mercer, Christopher Paul
(86) International application number: PCT/IB2004/001253
(87) International publication number: WO 2004/096174

(56) References cited:
- WO-A-01/70194
- WO-A-02/03956
- WO-A-03/030881
- US-A1- 2003 008 008

## Description

### Field of the Invention

The present invention is related generally to fast dissolving orally consumable films, more particularly to films containing a pharmaceutically active agent.

### Background of Related Technologies

Personal care products can be formulated in a variety of dosage forms, including tablets, capsules, lozenges or strips of edible thin film compositions. Edible thin film compositions applied to the oral cavity can be designed to deliver therapeutic agents to the oral mucosa. One such example is LISTERINE POCKETPAKS^{™} brand oral care strip products made by Pfizer Inc. of New York are successful examples of an edible film compositions effective in delivering therapeutic agents particularly antimicrobial agents in the form of a combination of essential oils.

US publication US 2003/0008008 A1 discloses a rapidly dissolvable, physiologically acceptable film comprising an antimicrobial effective amount of an essential oil. PCT publication WO 01/70194 A discloses fast dissolving orally consumable films containing a water soluble polymer, an active agent and a taste-masking agent. PCT publication WO 03/030881 A discloses an ingestible water-soluble film delivery system comprising a glucan and a water-soluble polymer.

Conventional rapidly dissolving orally consumable films absorb water and may become viscous and sticky over time when applied to the moist surface of the mucosa of the oral cavity. Retention of the film may be insufficient to obtain the desired effect because the film rapidly disintegrates within a relatively short time. Sometimes it is desirable to have improved covering and adherence to the mucosa surface. Thus, there is a need in the art to develop consumable thin films, having good adhesion and retention to the mucosa of the oral cavity for providing an effective delivery and retention system for antitussive and mucosa coating agents.

### Summary

The present invention is generally directed to a consumable film, which is particularly well adapted to rapidly dissolve in the mouth of a consumer. In one particular aspect of the present invention, there is provided a consumable film, as defined in claim 1, adapted to adhere to and dissolve in the mouth of a consumer comprising at least one water soluble polymer, at least one antitussive agent and a mucosa-coating effective amount of a mucosa-coating agent. The mucosa-coating agent is pectin. In another aspect of the invention, there is provided a consumable film adapted to adhere to and dissolve in the oral cavity of a consumer comprising at least one water soluble polymer, as defined in claim 1, and a pharmaceutically active agent selected from the group consisting of aspirin, acetaminophen, ibuprofen, ketoprofen, diflunisal, fenoprofen calcium, naproxen, tolmetin sodium, indomethacin, flurbiprofen sodium, celecoxib, valdecoxib, rofecoxib and mixtures thereof.

A method is disclosed for preparing a supple, non-self-adhering film especially suitable for oral delivery of at least one antitussive agent. The method comprises preparing an aqueous phase comprising a mucosa-coating effective amount of a mucosa-coating agent; preparing a film-forming mixture including at least one water soluble polymer; combining the aqueous phase and the film forming mixture to form a hydrated polymer gel; casting the hydrated polymer gel on a substrate to form a cast gel; and drying the cast gel to form the consumable film, wherein the at least one antitussive agent is added to the aqueous phase, the hydrated polymer gel or both.

### Detailed Description

The present invention is directed to a physiological acceptable consumable film, as defined in claim 1, that is adapted to dissolve in the mouth of a consumer and adhere to the mucosa of the oral cavity. Consumable films with mucosa coating agents are particularly well-suited for delivering an antitussive agent to the consumer and are useful for treating or alleviating the symptoms and/or irritations associated with sore throats and/or coughing.

In one aspect of the present invention, there is provided a consumable film, as defined in claim 1, adapted to adhere to and dissolve in the mouth of a consumer including at least one water soluble polymer, at least one antitussive agent and a mucosa-coating effective amount of mucosa-coating agent. The mucosa-coating agent is capable of forming a coating that adheres to the mucosa of the mouth and throat whereby the antitussive agent is effectively retained in contact with the affected areas of the mouth and throat for a period time after the consumable film has dissolved.

In another aspect of the invention, there is provided a consumable film, as defined in claim 1, adapted to adhere to and dissolve in the oral cavity of a consumer comprising at least one water soluble polymer and a pharmaceutically active agent selected from the group consisting of aspirin, acetaminophen, ibuprofen, ketoprofen, diflunisal, fenoprofen calcium, naproxen, tolmetin sodium, indomethacin, fluriprofen sodium, celecoxib, valdecoxib, rofecoxib and mixtures thereof. In one particular embodiment, the consumable film includes the pharmaceutically active agent which is valdecoxib in amounts from about 5 to about 20 milligrams.

The consumable film may include one or more of the following ingredients, including, but not limited to, water, antimicrobial agents, additional film forming agents or water soluble polymers, plasticizing agents, flavorings, sulfur precipitating agents, saliva stimulating agents, cooling agents, surfactants, stabilizing agents, emulsifying agents, thickening agents, binding agents, coloring agents, triglycerides, polyethylene oxides, propylene glycols, sweeteners, fragrances, preservatives and the like, as described in US publication US 2003/0054034 A1, by Leung et al., filed September 14, 1999.

In another embodiment of the present invention, the consumabe film, as defined in claim 1, comprises a single layer including at least one water soluble polymer, at least one antitussive agent and a mucosa-coating effective amount of pectin.

The term "consumable" as used herein is intended to emcompass substances including edible compounds, which upon administration to a consumer, is adequately tolerated without causing undue adverse effects or discomfort to the consumer.

Unless specified otherwise, the term "% by weight" as used is based on the total weight of the final product (i.e., the consumable film) as opposed to the formulation used to produce the product, and thus denotes the percent of the total dry weight contributed by the subject ingredient. This theoretical value can differ from the experimental value, because in practice, the consumable film typically retains some of the water and/or other substances such as alcohol (e.g. ethanol) that may be used in preparing the final product.

In one embodiment, the consumable film of the present invention, as defined in claim 1, is shaped and sized for administration to the oral cavity. The mucosa coating agent is capable of imparting throat soothing and throat coating properties to the consumable film as the film dissolves in the consumer's mouth. The dissolved film adheres to the surface of the mouth, typically the roof of the mouth or the tongue, and coats and adheres to the mucosa of the throat, thus providing maximum retention thereon for an extended period of time. As a result, the consumable film of the present invention affords an effective delivery and retention system for therapeutic agents to localized areas within the oral cavity for which treatment with the therapeutic agent is desired. Suitable mucosa-coating agents include pectin, gelatin, and combinations thereof. The mucosa-coating agent is present in amounts ranging from about 0.1% to about 2% by weight, and in another embodiment, from about 0.1% to about 1.0% by weight of the consumable film.

Suitable antitussive agent include alloclamide, amicibone, benproperine, benzonatate, bibenzonium bromide, bromoform, butamirate, butetamate, caramiphen ethanedisulfonate, caramiphen edisylate, carbetapentane, chlophedianol, clobutinol, cloperastine, codeine, codeine methyl bromide, codeine N-oxide, codeine phosphate, codeine sulfate, cyclexanone, dextromethorphan, dibunate sodium, dihydrocodeine, dihydrocodeinone enol acetate, dimemorfan, dimethoxanate, ∀,∀-diphenyl-2-piperidinepropanol, dropropizine, drotebanol, eprazinone, ethyl dibunate, ethylmorphine, fominoben, guaiapate, hydrocodone, isoaminile, levopropoxyphene, morclofone, narceine, normethadone, noscapine, oxeladin, oxolamine, pholcodine, picoperine, pipazethate, piperidione, prenoxdiazine hydrochloride, racemethorphan, taziprinone hydrochloride, tipepidine, zipeprol, and the like and pharmaceutically acceptable salts thereof, and combinations thereof. The antitussive agents as utilized in the present invention may be in the free form or in any non-toxic pharmaceutically acceptable form wherein their therapeutic activity is retained. In one embodiment, the antitussive agent is dextromethorphan hydrobromide.

The antitussive agent, whether a single antitussive agent or combinations thereof, is employed in an effective amount. An "effective amount" is an amount of the antitussive agent that is sufficient to at least reduce the occurrence of coughing and/or the adverse effects of a sore throat, but low enough to avoid any adverse side effects. In addition to the particular antitussive agent or agents chosen, the effective amount of the antitussive agent may vary with the type and/or severity of the coughing condition, the age and physical condition of the patient being treated, the duration of treatment, the type of concurrent therapy, the specific form (e.g., salt) of the antitussive agent employed, and the particular formulation of the consumable film which contains the antitussive agent. These variations can be readily determined by one of ordinary skill in the art.

The amount of antitussive agent is adjusted to deliver a predetermined dose of the antitussive agent over a predetermined period of time, which may typically vary from 4 to 24 hours, more typically about every 12 hours. A typical adult dose of an antitussive agent will contain from about 1 to about 130 mg, preferably from about 2.5 mg to about 65 mg, more preferably from about 2.5 to about 20 and most preferably about 15 mg of the antitussive agent (e.g., dextromethorphan hydrobromide). A typical child dose of an antitussive agent will contain from about 2.5 to about 10 mg and more preferably about 7.5 mg of dextromethorphan hydrobromide.

Except as otherwise noted, the amount of antitussive agent in the consumable film according to the present invention is designated as % by weight after the "wet" film formulation has been dried and formed into the consumable film. Generally, the amount of the antitussive agent used in the consumable film is from about 0.01% to about 80% by weight based on the total weight of the consumable film, preferably from about 2.5% to about 40% by weight, and more preferably from about 5% to about 30% by weight.

A film can measure from about 1" by about 1.25" (2.54 cm x 3.18 cm) and weigh from about 60 mg to about 190 mg.

Additional therapeutic agents that are effective for treating conditions other than coughing may be added to various embodiments of the present invention, such as an antihistamine, symphathomimetic pharmaceutically active agent (nasal decongestant, bronchodilator), analgesic, anti-inflammatory, cough expectorant and the like, as described in US publication US 2003/0054034 A1, by Leung et al., filed September 14, 1999. Other examples of such additional therapeutic agents are well known in the art.

Useful antihistamines include cetirizine, diphenhydramine, loratadine, desloratadine, fexofenadine, montelukast sodium, and the like.

Examples of doses for specific pharmaceutically active agents that can be delivered per one strip of rapidly dissolving oral film are reviewed in Table A.

**Table A**

| Pharmaceutically Active Agent | Dose |
|---|---|
| Chlorpheniramine Maleate | 4-12 mg |
| Brompheniramine Maleate | 4 mg |
| Dexchlorpheniramine | 2 mg |
| Dexbropheniramine | 2 mq |
| Triprolidine Hydrochloride | 2.5 mg |
| Cetirizine | 5-10 mg |
| Acrivastine | 8 mg |
| Azatadine Maleate | 1 mg |
| Loratadine | 5-10 mg |
| Phenylephrine Hydrochloride | 5-10 mg |
| Dextromethorphan Hydrobromide | 10-30 mg |
| Sildenafil | 25-100 mg |
| Ketoprofen | 12.5-25 mg |
| Sumatriptan Succinate | 35-70 mg |
| Zolmitriptan | 2.5 mg |
| Loperamide | 2 mg |
| Famotidine | 5-10 mg |
| Nicotine | 1-15 mg |
| Diphenhydramine Hydrochloride | 12.5-25 mg |
| Pseudoephedrine Hydrochloride | 15-60 mg |
| Atorvastatin | 5-80 mg |
| Valdecoxib | 5-20 mg |
| Amlodipine besylate | 2.5-10 mg |
| Rofecoxib | 5-25 mg |
| Setraline hydrochloride | 10-100 mg |
| Ziprasidone | 20-80 mg |
| Eletriptan | 10-40 mg |
| Nitroglycerin | 0.3-0.6 mg |

The film compositions of the present invention may also be used to supply nutritionally acceptable components such as vitamins, minerals, trace elements, and fibers (preferably soluble fibers).

Examples of vitamins suitable for the incorporation in the composition of the invention include Vitamin A, Vitamin D, Vitamin E, Vitamin K, Vitamin C, folic acid, thiamin, riboflavin, Vitamin B (6), Vitamin B (12), niacin, biotin and panthotenic acid in pharmaceutical or nutritionally acceptable form. Examples of mineral elements and trace elements suitable for the incorporation in the composition of the invention include calcium, sodium, potassium, phosphorous, magnesium, manganese, copper, zinc, iron, selenium, chromium and molybdenum in pharmaceutical or nutritionally acceptable form.

The term soluble fiber as used herein refers to fibers which are able to substantially undergo fermentation in the colon to produce short chain fatty acids. Examples of suitable soluble fibers include, carubin, pectin, tragacanth, cereal betaglucan and the like. They may be hydrolysed or not.

Useful water soluble polymers that exhibit film forming properties include pullulan, hydroxypropylmethyl cellulose, hydroxyethyl cellulose, hydroxypropyl cellulose, polyvinyl pyrrolidone, carboxymethyl cellulose, polyvinyl alcohol, sodium alginate, polyethylene glycol, tragacanth gum, guar gum, acacia gum, arabic gum, polyacrylic acid, methylmethacrylate copolymers, carboxyvinyl polymers, amylose, high amylose starch, hydroxypropylated high amylose starch, dextrin, chitin, chitosan, levan, elsinan, collagen, zein, gluten, soy protein isolate, whey protein isolate, casein and combinations thereof. In one embodiment of the present invention the film, as defined in claim 1, comprises pullulan as a water soluble polymer. The amount of the water soluble polymer will typically be from about 0.01% to about 99% by weight, preferably from about 30% to about 80% by weight, more preferably from about 45% to about 70% by weight of the consumable film and most preferably from about 60% to about 65% by weight of the consumable film.

In another embodiment of the present invention, the consumable film, as defined in claim 1, may further include antimicrobial agents including, but not limited to, essential oils as is described in US publication US 2003/0054034 A1, by Leung et al., filed September 14, 1999. Useful essential oils carvacrol, thymol, eucalyptol, menthol, methyl salicylate, eugenol, gerianol, verbenone, and the like and combinations thereof. One of the preferred combinations of essential oils for use in the present invention is utilized in LISTERINE® brand mouthwash and oral care strips, which is a well known example of antiseptic oral composition that has proven effective in killing microorganisms in the oral cavity contribute to the formation of plaque, gingivitis and bad breath. Essential oils include precisely balanced amounts of thymol, methyl salicylate, menthol and eucalyptol (hereinafter "the preferred essential oils") having antimicrobial activity.

The amounts of the essential oils used in the consumable film of the present invention can vary as long as they are in amounts sufficient to provide antimicrobial efficacy. Generally, the amount of essential oils is up to about 30% and preferably from about 0.05% to about 18% by weight of the consumable film. In one preferred embodiment, the amount of thymol, methyl salicylate and eucalyptol is each from about 0.01% to about 4% by weight, preferably from about 0.05% to about 3.0% by weight and more preferably from about 0.07% to about 2.0% by weight of the consumable film. Menthol may be present in an amount of from about 0.01% to about 15% by weight of the composition, preferably from about 2.0% to about 9.0% by weight and more preferably from about 3% to about 9% by weight of the consumable film. In certain embodiments, the essential oils are combined in amounts to provide synergistically enhanced antiseptic properties to eradicate plaque-producing germs that cause dental plaque, gingivitis and bad breath.

For embodiments incorporating essential oils, humectants are avoided due to the relatively high content of oil in the consumable, so as to avoid producing an overly moist, self-adhering film. In an embodiment, the consumable film includes a plasticizing agent other than glycerin, which is also a humectant, and with a sweetener other than sorbitol, which is a mild humectant.

Saliva stimulating agents may also be added to the consumable films of the present invention. Useful saliva stimulating agents are disclosed in U.S. Pat. No. 4,820,506, which is incorporated herein by reference in its entirety.

Suitable sweeteners include both natural and artificial sweeteners such as A) water-soluble sweeteners including monosaccharides, disaccharides, polysaccharides and the like, B) water-soluble artificial sweeteners including soluble saccharin salts and the like, C) dipeptide based sweeteners such as L-aspartic acid derived sweeteners including aspartame, neotame and the like, D) derivatives of naturally occurring water-soluble sweeteners including chlorinated derivatives of sucrose, sucralose and the like, E) protein based sweeteners including thaumatoccous danielli (Thaumatin I and II) and the like, and combinations thereof.

In general, an effective amount of auxiliary sweetener is utilized to provide the level of sweetness desired for a particular composition, and this amount will vary with the particular sweetener selected. The effective amount will normally be from about 0.01% to about 10% by weight of the consumable film when using an easily extractable sweetener. The water-soluble sweeteners are usually used in amounts of from about 0.01% to about 10% by weight, and preferably in amounts of from about 2.0% to about 5.0% by weight of the consumable film. The other sweeteners described above, other than water-soluble sweeteners are generally used in amounts of from about 0.01% to about 10% by weight, preferably from about 2% to about 8% by weight, and more preferably from about 3% to about 6% by weight of the consumable film.

A preservative may also be added to the consumable films. The preservative is added in amounts from about 0.001 % to about 5%, preferably from about 0.01 % to about 1 % by weight of the consumable film. Preferred preservatives include sodium benzoate, potassium sorbate and the like, and combinations thereof. Other suitable preservatives include, but are not limited to, salts of edetate, (also known as salts of ethylenediaminetetraacetic acid, or EDTA, such a disodium EDTA).

Also disclosed are methods of preparing consumable films of the present invention. Generally, at least one antitussive agent and a mucosa-coating effective amount of a mucosa-coating agent are dissolved in water to form an aqueous phase. The aqueous phase may further include sweeteners, dyes, and the like. A film forming mixture comprising at least one water soluble polymer (e.g., pullulan) is prepared. The aqueous phase and the film forming mixture are combined and thoroughly mixed to form a hydrated polymer gel. Optionally, an organic phase comprising organic ingredients such as essential oils and other oils (e.g. glycerine, olive oil) flavorants, surfactants (e.g., Polysorbate 80, Atmos 300, Atsurf 596K); and the like, may be combined with the aqueous phase, the film forming mixture or the hydrated polymer gel. The resulting hydrated polymer gel is cast on a suitable substrate to form a cast gel. The cast gel is then dried to form the consumable film.

In a further disclosed method of preparing the consumable film, it may be desirable to first form the film forming mixture by first hydrating the water soluble polymer with water. The aqueous phase is then prepared by dissolving the other water soluble ingredients such as the antitussive agent, the mucosa-coating agent (e.g., pectin), sweeteners, dyes, and the like in water. Separately, the organic ingredients such as essential oils and other oils (e.g. glycerine, olive oil) flavorants, surfactants (e.g., Polysorbate 80, Atmos 300, Atsurf 596K); and the like are mixed together. The final formulation is then produced by mixing the film forming polymer phase with the aqueous phase, then adding the organic phase. The combined mixture is formed into an emulsion or a hydrated polymer gel.

The resulting hydrated polymer gel is then cast on a suitable substrate and dried to form a film. The consumable film is preferably air-dried and dried under warm air and cut to a desired dimension, packaged and stored. The packaged film may contain moisture in amounts of from about 0.1% to about 10% by weight, and more preferably from about 4% to about 7% by weight.

The film forming mixture further includes stabilizing agents selected from xanthan gum, locust bean gum, carrageenan, and combinations thereof. These ingredients are mixed and then hydrated in warm water, preferably deionized water until a gel is formed which may take from about 30 to about 48 hours. The water is preferably heated to a temperature of from about 20ºC to about 40ºC to promote hydration. The amount of water is typically from about 40% to about 80% by weight of the gel. The resulting hydrated gel is then chilled to a temperature of from about 20ºC to about 30ºC for about 1 hour to about 48 hours.

The aqueous phase may, in addition to the antitussive agent and the mucosa coating effective amount of the mucosa-coating agent such as pectin, include additives such as coloring agents, copper gluconate and sweetener. Typically the aqueous phase contains from about 5% to about 80% by weight based on the total weight of the final gel mixture.

If sodium saccharin as a selected sweetener and copper gluconate as a selected sulfur precipitating agent are used in the formulation, it is preferable to dissolve them separately in solution to avoid precipitation.

In another disclosed method, the water soluble polymer is in the form of a powder which is added to the aqueous phase to form a hydrated polymer gel. The resulting hydrated polymer gel is thoroughly stirred at about room temperature for about 30 minutes to about 48 hours, and then deaerated to remove at least substantially all the air bubbles. The uniform mixture is cast on a suitable substrate, and thereafter dried to form the desired film.

For consumable films containing essential oils, the essential oils are further added to the organic phase and the mixing the organic phase with the hydrated polymer gel. In particular, the essential oils such as menthol and thymol can be mixed optionally in combination with oils to form an oil mixture. Other essentials oils such as methyl salicylate and eucalyptol, and surfactants can then be added to the oil mixture. The oil mixture is then added to the hydrated polymer gel and mixed until a uniform gel is formed. The uniform gel is then cast on a suitable substrate, and thereafter dried to form the consumable film.

In one disclosed method for preparing the consumable film, the water soluble polymer may be hydrated without heating the water to reduce energy costs in the manufacturing process. Moreover, since heating may result in undesirable losses of volatile ingredients to evaporation, it would be preferable to avoid heating during the hydration process. For essential oil-containing films, the heat may also affect the germ killing activity of the composition due to the loss of essential oils.

While not wishing to be bound by any theory, it is believed that the film forming ingredients such as the water soluble polymers can be hydrated and mixed without heating due to an ionic effect known as the Donnan equilibrium. Hydrating the water soluble polymers in the presence of electrolytes in solution effectively lowers the viscosity of the polymer gel being formed, thus increasing the efficiency of the hydrating process. The water-soluble ingredients of the formulation provide the electrolytes, which are dissolved in the hydration solution prior to addition to the water-soluble polymers. High shear mixing also accelerates hydration, which delumps the powders, providing greater surface area for water contact. In addition, local heating effects, generated in the shear regions, provide energy for hydration without substantially raising the temperature of the mass.

### EXAMPLE 1

The ingredients listed in Table 1 were combined to provide a consumable film of the present invention in accordance with the following procedure:
A) Dextromethorphan HBr was mixed and dissolved in 90% water at 75ºC to yield an aqueous phase. Amberlite IRP69 was added to the aqueous phase and stirred for about 4 to 5 hours at about 70ºC to 80ºC. Pectin was added to the aqueous phase very slowly and mixed at high speed. The aqueous phase was allowed to cool to about 50ºC and q.s. with water to replace loss due to evaporation. Potassium sorbate, sweeteners and dye were then added to the aqueous phase and mixed thoroughly.
B) The film-forming ingredients, xanthan gum, locust bean gum, carrageenan and pullulan were mixed together in a separate container to form a film forming mixture.
C) The film forming mixture was slowly added to the aqueous phase of A), followed by overnight mixing at a slow rate to provide a hydrated polymer gel.
D) The flavorants, glycerine, menthol, and surfactants were combined and mixed in a separate container until dissolved to yield an organic phase.
E) Mannitol was mixed together in the remaining 10% water in a separate container. Succulence was then added to the resulting mixture and dissolved.
F) The mixtures of steps D) and E) were added to the hydrated polymer gel and mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel). The consumable film was segmented into 1" x 1.25" (2.54 cm x 3.18 cm) dosage units, each of which had a thickness of 0.009 ± 0.002 of an inch (0.23 ± 0.05 of a mm) and a weight of 70 ± 3 mg.

**Table 1**

| Material | mg/dose* | %w/w* Dry Film | %w/w Actual Batch | g/batch |
|---|---|---|---|---|
| Dextromethorphan HBr | 15.0000 | 22.3940 | 7.6539 | 38.2695 |
| Amberlite IRP69 | 16.0000 | 23.8870 | 8.1642 | 40.8208 |
| Pectin USP | 0.3500 | 0.5225 | 0.1786 | 0.8930 |
| Xanthan Gum | 0.0766 | 0.1165 | 0.0396 | 0.1980 |
| Locust Bean Gum | 0.0901 | 0.1345 | 0.0460 | 0.2299 |
| Carrageenan | 0.3861 | 0.5764 | 0.1970 | 0.9851 |
| Pullulan | 20.5919 | 30.7424 | 10.5072 | 52.5361 |
| Potassium sorbate | 0.0772 | 0.1153 | 0.0394 | 0.1970 |
| Acesulfame Potassium salt | 0.6435 | 0.9607 | 0.3284 | 1.6418 |
| Aspartame NF | 1.8018 | 2.6900 | 0.9194 | 4.5969 |
| Purified water | - | - | 65.8217 | 329.1085 |
| Menthol | 2.5740 | 3.8428 | 1.3134 | 6.5670 |
| Peppermint Flavor | 0.2579 | 0.3850 | 0.1316 | 0.6580 |
| Cherry Flavor (Givudan) | 0.2579 | 0.3850 | 0.1316 | 0.6580 |
| Cherry Flavor Blend (IFF) | 2.2350 | 3.3367 | 1.1404 | 5.7022 |
| Warm Sensation (Mane) | 0.5518 | 0.8238 | 0.2816 | 1.4078 |
| Artificial Masking Agent Flavor (Robertet) | 0.4139 | 0.6179 | 0.2112 | 1.0560 |
| Succulence (IFF) | 0.2579 | 0.3850 | 0.1316 | 0.6580 |
| FD&C Red #40 | 0.0102 | 0.0152 | 0.0052 | 0.0260 |
| Polysorbate 80 NF | 0.4504 | 0.6724 | 0.2298 | 1.1491 |
| Atmos 300 | 0.4504 | 0.6724 | 0.2298 | 1.1491 |
| Glycerine | 1.9305 | 2.8821 | 0.9851 | 4.9253 |
| Mannitol USP | 2.5740 | 3.8428 | 1.3134 | 6.5670 |
| Total | 66.9821 | 100.0000 | 100.0000 | 500.0000 |
| *Assuming that all water is evaporated | | | | |

### EXAMPLE 2

The ingredients listed in Table 2 were combined to provide a consumable film of the present invention in accordance with the following procedure:
A) Dextromethorphan HBr was mixed and dissolved in 90% water at 75ºC to yield an aqueous phase. Amberlite IRP64 was added to the aqueous phase and stirred for about 4 to 5 hours at about 70ºC to 80ºC. Pectin was mixed with glycerine and the mixture was added very slowly to the aqueous phase and then mixed thoroughly at a high rate. The aqueous phase was allowed to cool to about 50ºC and q.s. with water to replace loss due to evaporation. Potassium sorbate and dye were then added to the aqueous phase and mixed thoroughly.
B) The film-forming ingredients, xanthan gum, locust bean gum, carrageenan and pullulan were mixed together in a separate container to form a film forming mixture.
C) The film forming mixture was slowly added to the aqueous phase of A), followed by overnight mixing at a slow rate to provide a hydrated polymer gel.
D) The flavorants and menthol were combined and mixed in a separate container until dissolved to yield an organic phase.
E) Mannitol and sucralose were mixed together in the remaining 10% water in a separate container. Succulence was then added to the resulting mixture and dissolved.
F) The mixtures of steps D) and E) were added to the hydrated polymer gel and mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel). The consumable film was segmented into 1" x 1.25" (2.54 cm x 3.18 cm) dosage units, each of which had a thickness of 0.009 ± 0.002 of an inch (0.23 ± 0.05 of a mm) and a weight of 70 ± 3 mg.

**Table 2**

| Material | mg/dose* | %w/w* Dry Film | %w/w Actual Batch | g/batch |
|---|---|---|---|---|
| Dextromethorphan HBr | 15.0000 | 22.9235 | 7.8353 | 39.1765 |
| Amberlite IRP64 | 16.0000 | 24.4518 | 8.3576 | 41.7882 |
| Pectin USP | 0.3500 | 0.5349 | 0.1828 | 0.9141 |
| Xanthan Gum | 0.0769 | 0.1175 | 0.0402 | 0.2008 |
| Locust Bean Gum | 0.0901 | 0.1377 | 0.0471 | 0.2353 |
| Carrageenan | 0.3861 | 0.5901 | 0.2017 | 1.0084 |
| Pullulan | 20.5919 | 31.4693 | 10.7562 | 53.7812 |
| Potassium sorbate | 0.0772 | 0.1180 | 0.0403 | 0.2016 |
| Purified water | - | - | 65.8199 | 329.0995 |
| Menthol | 2.5740 | 3.9337 | 1.3445 | 6.7227 |
| Peppermint Flavor | 0.2579 | 0.3941 | 0.1347 | 0.6736 |
| Cherry Flavor (Givudan) | 0.2579 | 0.3941 | 0.1347 | 0.6736 |
| Sour Cherry (IFF) | 2.2350 | 3.4156 | 1.1675 | 5.8373 |
| Warm Sensation (Mane) | 0.5518 | 0.8433 | 0.2882 | 1.4412 |
| Artificial Masking Agent Flavor (Robertet) | 0.4139 | 0.6325 | 0.2162 | 1.0810 |
| Succulence (IFF) | 0.2579 | 0.3941 | 0.1347 | 0.6736 |
| FD&C Red #40 | 0.0098 | 0.0150 | 0.0051 | 0.0256 |
| Glycerine | 1.9305 | 2.9503 | 1.0084 | 5.0420 |
| Mannitol USP | 2.5740 | 3.9337 | 1.3445 | 6.7227 |
| Sucralose | 1.8000 | 2.7508 | 0.9402 | 4.7012 |
| | | | | |
| Total | 65.4349 | 100.0000 | 100.0000 | 500.0000 |
| *Assuming that all water is evaporated | | | | |

### EXAMPLE 3

The ingredients listed in Table 3 were combined to provide a consumable film of the present invention in accordance with the procedure of Example 1.

**Table 3**

| Material | mg/dose* | %w/w* Dry Film | %w/w Actual Batch | g/batch |
|---|---|---|---|---|
| Dextromethorphan HBr | 15.0000 | 22.6123 | 7.7289 | 38.6445 |
| Amberlite IRP69 | 16.0000 | 24.1197 | 8.2442 | 41.2208 |
| Pectin USP | 0.3500 | 0.5276 | 0.1803 | 0.9017 |
| Xanthan Gum | 0.0769 | 0.1159 | 0.0396 | 0.1981 |
| Locust Bean Gum | 0.0901 | 0.1358 | 0.0464 | 0.2321 |
| Carrageenan | 0.3861 | 0.5820 | 0.1989 | 0.9947 |
| Pullulan | 20.5919 | 31.0420 | 10.6102 | 53.0509 |
| Potassium sorbate | 0.0772 | 0.1164 | 0.0398 | 0.1989 |
| Purified water | - | - | 65.8199 | 329.0995 |
| Menthol | 2.5740 | 3.8803 | 1.3263 | 6.6314 |
| Peppermint Flavor | 0.2579 | 0.388 | 0.1329 | 0.6644 |
| Cherry Flavor (Givudan) | 0.2579 | 0.388 | 0.1329 | 0.6644 |
| Cherry Flavor Blend (IFF) | 2.2350 | 3.3692 | 1.1516 | 5.7580 |
| Warm Sensation (Mane) | 0.5518 | 0.8318 | 0.2843 | 1.4216 |
| Artificial Masking Agent Flavor (Robertet) | 0.4139 | 0.6239 | 0.2133 | 1.0663 |
| Succulence (IFF) | 0.2579 | 0.3888 | 0.1329 | 0.6644 |
| FD&C Red #40 | 0.0098 | 0.0148 | 0.0050 | 0.0252 |
| Polysorbate 80 NF | 0.4504 | 0.6790 | 0.2321 | 1.1604 |
| Atmos 300 | 0.4504 | 0.6790 | 0.2321 | 1.1604 |
| Glycerine | 1.9305 | 2.9102 | 0.9947 | 4.9735 |
| Mannitol USP | 2.5740 | 3.8803 | 1.3263 | 6.6314 |
| Sucralose | 1.8000 | 2.7135 | 0.9275 | 4.6373 |
| | | | | |
| | | | | |
| Total | 66.3357 | 100.0000 | 100.0000 | 500.0000 |
| *Assuming that all water is evaporated | | | | |

### EXAMPLE 4

The ingredients listed in Table 4 were combined to provide a consumable film of the present invention in accordance with the procedure of Example 2, except glycerine and surfactants were also added to the flavorants and menthol in step D).

**Table 4**

| Material | mg/dose* | %w/w* Dry Film | %w/w Actual Batch | g/batch |
|---|---|---|---|---|
| Dextromethorphan HBr | 15.0000 | 22.6123 | 7.7289 | 38.6445 |
| Amberlite IRP64 | 16.0000 | 24.1197 | 8.2442 | 41.2208 |
| Pectin USP | 0.3500 | 0.5276 | 0.1803 | 0.9017 |
| Xanthan Gum | 0.0769 | 0.1159 | 0.0396 | 0.1981 |
| Locust Bean Gum | 0.0901 | 0.1358 | 0.0464 | 0.2321 |
| Carraqeenan | 0.3861 | 0.5820 | 0.1989 | 0.9947 |
| Pullulan | 20.5919 | 31.0420 | 10.6102 | 53.0509 |
| Potassium sorbate | 0.0772 | 0.1164 | 0.0398 | 0.1989 |
| Purified water | - | - | 65.8199 | 329.0995 |
| Menthol | 2.5740 | 3.8803 | 1.3263 | 6.6314 |
| Peppermint Flavor | 0.2579 | 0.3888 | 0.1329 | 0.6644 |
| Cherry Flavor (Givudan) | 0.2579 | 0.3888 | 0.1329 | 0.6644 |
| Sour Cherry (IFF) | 2.2350 | 3.3692 | 1.1516 | 5.7580 |
| Warm Sensation (Mane) | 0.5518 | 0.8318 | 0.2843 | 1.4216 |
| Artificial Masking Agent Flavor (Robertet) | 0.4139 | 0.6239 | 0.2133 | 1.0663 |
| Succulence (IFF) | 0.2579 | 0.3888 | 0.1329 | 0.6644 |
| FD&C Red #40 | 0.0098 | 0.0148 | 0.0050 | 0.0252 |
| Polysorbate 80 NF | 0.4504 | 0.6790 | 0.2321 | 1.1604 |
| Atmos 300 | 0.4504 | 0.6790 | 0.2321 | 1.1604 |
| Glycerine | 1.9305 | 2.9102 | 0.9947 | 4.9735 |
| Mannitol USP | 2.5740 | 3.8803 | 1.3263 | 6.6314 |
| Sucralose | 1.8000 | 2.7135 | 0.9275 | 4.6373 |
| | | | | |
| | | | | |
| Total | 66.3357 | 100.0000 | 100.0000 | 500.0000 |
| *Assuming that all water is evaporated | | | | |

### EXAMPLE 5

The ingredients listed in Table 5 were combined to provide a consumable film of the present invention in accordance with the following procedure:
A) Dextromethorphan HBr was mixed and dissolved in 90% water at 75ºC to yield an aqueous phase. Amberlite IRP69 was added to the aqueous phase and stirred for about 4 to 5 hours at about 70ºC to 80ºC. Pectin was added to the aqueous phase very slowly and mixed at a high mixing rate. The aqueous phase was allowed to cool to about 50ºC and q.s. with water to replace loss due to evaporation. Potassium sorbate and dye were then added to the aqueous phase and mixed thoroughly.
B) The film-forming ingredients, xanthan gum, locust bean gum, carrageenan and PURE-COTE^{™} B793 (available from Grain Processing Corporation of Muscatine, lowa) were mixed together in a separate container to form a film forming mixture.
C) The film forming mixture was slowly added to the aqueous phase of A), followed by overnight mixing at a low mixing rate to provide a hydrated polymer gel.
D) The flavorants, glycerine, olive oil, menthol, and surfactants were combined and mixed in a separate container until dissolved to yield an organic phase.
E) Mannitol and sucralose were mixed together in the remaining 10% water in a separate container. Succulence was then added to the resulting mixture and dissolved.
F) The mixtures of steps D) and E) were added to the hydrated polymer gel and mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel). The consumable film was segmented into 1" x 1.25" (2.54 cm x 3.18 cm) dosage units, each of which had a thickness of 0.009 ± 0.002 of an inch (0.23 ± 0.05 of a mm) and a weight of 70 ± 3 mg.

**Table 5**

| Material | mg/dose* | %w/w* Dry Film | %w/w Actual Batch | g/batch |
|---|---|---|---|---|
| Dextromethorphan HBr | 15.0000 | 19.5740 | 10.6759 | 106.7593 |
| Amberlite IRP69 | 16.0001 | 20.8790 | 11.3877 | 113.8771 |
| Pectin USP | 0.3499 | 0.4566 | 0.2490 | 2.4905 |
| Xanthan Gum | 0.0769 | 0.1003 | 0.0547 | 0.5470 |
| Locust Bean Gum | 0.0901 | 0.1175 | 0.0641 | 0.6409 |
| Carrageenan | 0.3860 | 0.5037 | 0.2747 | 2.7474 |
| PURE-COTE^{™} B793 | 20.5919 | 26.8711 | 14.6559 | 146.5586 |
| Potassium sorbate | 0.0772 | 0.1008 | 0.0550 | 0.5498 |
| Purified water | - | - | 45.4586 | 454.5856 |
| Menthol | 2.5740 | 3.3589 | 1.8320 | 18.3202 |
| Peppermint Flavor | 0.2579 | 0.3366 | 0.1836 | 1.8357 |
| Cherry Flavor (Givudan) | 0.2579 | 0.3366 | 0.1836 | 1.8357 |
| Sour Cherry (IFF) | 2.2350 | 2.9165 | 1.5907 | 15.9070 |
| Warm Sensation (Mane) | 0.5518 | 0.7200 | 0.3927 | 3.9270 |
| Artificial Masking Agent Flavor (Robertet) | 0.4140 | 0.5402 | 0.2946 | 2.9463 |
| Succulence (IFF) | 0.2579 | 0.3366 | 0.1836 | 1.8357 |
| FD&C Red #40 | 0.0099 | 0.0129 | 0.0070 | 0.0704 |
| Polysorbate 80 NF | 0.4505 | 0.5878 | 0.3206 | 3.2060 |
| Atmos 300 | 0.4505 | 0.5878 | 0.3206 | 3.2060 |
| Glycerine | 8.7335 | 11.3966 | 6.2158 | 62.1585 |
| Olive Oil | 3.49934 | 4.5586 | 2.4863 | 24.8634 |
| Mannitol USP | 2.5740 | 3.3589 | 1.8320 | 18.3202 |
| Sucralose | 1.8001 | 2.3490 | 1.2812 | 12.8116 |
| | | | | |
| | | | | |
| Total | 76.6324 | 100.0000 | 100.0000 | 1000.0000 |
| *Assuming that all water is evaporated | | | | |

### EXAMPLE 6

The ingredients listed in Table 6 were combined to provide a consumable film of the present invention in accordance with the procedure of Example 5 except pectin was dispersed in 15% glycerine prior to being added to the aqueous phase in Step A).

**Table 6**

| Material | mg/dose* | %w/w* Dry Film | %w/w Actual Batch | g/batch |
|---|---|---|---|---|
| Dextromethorphan HBr | 15.0000 | 18.5409 | 10.3611 | 103.6107 |
| Amberlite IRP69 | 16.0001 | 19.7771 | 11.0519 | 110.5186 |
| Pectin USP | 0.3499 | 0.4325 | 0.2417 | 2.4170 |
| Xanthan Gum | 0.0769 | 0.0950 | 0.0531 | 0.5309 |
| Locust Bean Gum | 0.0901 | 0.1113 | 0.0622 | 0.6220 |
| Carrageenan | 0.3860 | 0.4771 | 0.2666 | 2.6664 |
| PURE-COTE^{™} B793 | 20.5919 | 25.4529 | 14.2236 | 142.2363 |
| Potassium sorbate | 0.0772 | 0.0955 | 0.0534 | 0.5335 |
| Purified water | - | - | 44.1179 | 451.1788 |
| Menthol | 2.5740 | 3.1817 | 1.7780 | 17.7799 |
| Peppermint Flavor | 0.2579 | 0.3188 | 0.1782 | 1.7816 |
| Cherry Flavor (Givudan) | 0.2579 | 0.3188 | 0.1782 | 1.7816 |
| Sour Cherry (IFF) | 2.2350 | 2.7626 | 1.5438 | 15.4379 |
| Warm Sensation (Mane) | 0.5518 | 0.6820 | 0.3811 | 3.8112 |
| Artificial Masking Agent Flavor (Robertet) | 0.4140 | 0.5117 | 0.2859 | 2.8594 |
| Succulence (IFF) | 0.2579 | 0.3188 | 0.1782 | 1.7816 |
| FD&C Red #40 | 0.0099 | 0.0122 | 0.0068 | 0.0684 |
| Polysorbate 80 NF | 0.4505 | 0.5568 | 0.3111 | 3.1114 |
| Atmos 300 | 0.4505 | 0.5568 | 0.3111 | 3.1114 |
| Glycerine | 11.6446 | 14.3935 | 8.0434 | 80.4337 |
| Olive Oil | 4.8519 | 5.9973 | 3.3514 | 33.5140 |
| Mannitol USP | 2.5740 | 3.1817 | 1.7780 | 17.7799 |
| Sucralose | 1.8001 | 2.2250 | 1.2434 | 12.4337 |
| | | | | |
| | | | | |
| Total | 80.9021 | 100.0000 | 100.0000 | 1000.0000 |
| *Assuming that all water is evaporated | | | | |

### EXAMPLE 7

The ingredients listed in Table 7 were combined to provide a consumable film of the present invention in accordance with the following procedure:
A) Dextromethorphan HBr was mixed and dissolved in 90% water at 75ºC to yield an aqueous phase. Amberlite IRP69 was added to the aqueous phase and stirred for about 4 to 5 hours at about 70ºC to 80ºC. Pectin dispersed in glycerine was added very slowly to the aqueous phase and mixed at a high mixing rate. The aqueous phase was allowed to cool to about 50ºC and q.s. with water to replace loss due to evaporation. The dye was then added to the aqueous phase and mixed thoroughly.
B) The film-forming ingredients, xanthan gum, locust bean gum, carrageenan and pullulan were mixed together in a separate container to form a film forming mixture.
C) The film forming mixture was slowly added to the aqueous phase of A), followed by overnight mixing at a low mixing rate to provide a hydrated polymer gel.
D) The flavorants, menthol, and surfactants were combined and mixed in a separate container until dissolved to yield an organic phase.
E) Mannitol and sucralose were mixed together in the remaining 10% water in a separate container. Succulence was then added to the resulting mixture and dissolved.
F) The mixtures of steps D) and E) were added to the hydrated polymer gel and mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel).

**Table 7**

| Material | mg/dose* | %w/w* Dry Film | %w/w Actual Batch | G/batch |
|---|---|---|---|---|
| Dextromethorphan HBr | 15.0000 | 22.5510 | 7.7080 | 19.2699 |
| Amberlite IRP64 | 16.0000 | 24.0544 | 8.2218 | 20.5545 |
| Pectin USP | 0.3500 | 0.5262 | 0.1799 | 0.4496 |
| Xanthan Gum | 0.0769 | 0.1156 | 0.0395 | 0.0988 |
| Locust Bean Gum | 0.0901 | 0.1355 | 0.0463 | 0.1157 |
| Carrageenan | 0.3861 | 0.5805 | 0.1984 | 0.4960 |
| Pullulan | 20.5919 | 30.9579 | 10.5814 | 26.4536 |
| Potassium sorbate | 0.0772 | 0.1161 | 0.0397 | 0.0992 |
| Purified water | - | - | 65.8199 | 164.5498 |
| Menthol | 2.5740 | 3.8698 | 1.3227 | 3.3067 |
| Peppermint Flavor | 0.2579 | 0.3877 | 0.1325 | 0.3313 |
| Cherry Flavor (Givudan) | 0.2579 | 0.3877 | 0.1325 | 0.3313 |
| Sour Cherry (IFF) | 2.2350 | 3.3601 | 1.1485 | 2.8712 |
| Warm Sensation (Mane) | 0.5518 | 0.8296 | 0.2835 | 0.7089 |
| Artificial Masking Agent Flavor (Robertet) | 0.4139 | 0.6223 | 0.2127 | 0.5317 |
| Succulence (IFF) | 0.2579 | 0.3877 | 0.1325 | 0.3313 |
| Carmine | 0.1900 | 0.2856 | 0.0976 | 0.2441 |
| Polysorbate 80 NF | 0.4504 | 0.6771 | 0.2314 | 0.5786 |
| Atsurf 596K | 0.4504 | 0.6771 | 0.2314 | 0.5786 |
| Glycerine | 1.9305 | 2.9023 | 0.9920 | 2.4800 |
| Mannitol USP | 2.5740 | 3.8698 | 1.3227 | 3.3067 |
| Sucralose | 1.8000 | 2.7061 | 0.9250 | 2.3124 |
| | | | | |
| | | | | |
| Total | 66.5159 | 100.0000 | 100.0000 | 250.0000 |
| *Assuming that all water is evaporated | | | | |

### EXAMPLE 8

The ingredients listed in Table 8 were combined to provide a consumable film of the present invention in accordance with the procedure of Example 7.

**Table 8**

| Material | mg/dose* | %w/w* Dry Film | %w/w Actual Batch | g/batch |
|---|---|---|---|---|
| Dextromethorphan HBr | 15.0000 | 22.5772 | 7.7169 | 38.5846 |
| Amberlite IRP64 | 16.0000 | 24.0823 | 8.2314 | 41.1569 |
| Pectin USP | 0.3500 | 0.5268 | 0.1801 | 0.9003 |
| Xanthan Gum | 0.0769 | 0.1157 | 0.0396 | 0.1978 |
| Locust Bean Gum | 0.0901 | 0.1356 | 0.0464 | 0.2318 |
| Carrageenan | 0.3861 | 0.5811 | 0.1986 | 0.9932 |
| Pullulan | 20.5919 | 30.9938 | 10.5937 | 52.9686 |
| Carmine | 0.1900 | 0.2860 | 0.0977 | 0.4887 |
| Purified water | - | - | 65.8199 | 329.0995 |
| Menthol | 2.5740 | 3.8742 | 1.3242 | 6.6211 |
| Peppermint Flavor | 0.2579 | 0.3882 | 0.1327 | 0.6634 |
| Cherry Flavor (Givudan) | 0.2579 | 0.3882 | 0.1327 | 0.6634 |
| Sour Cherry (IFF) | 2.2350 | 3.3640 | 1.1498 | 5.7491 |
| Warm Sensation (Mane) | 0.5518 | 0.8305 | 0.2839 | 1.4194 |
| Artificial Masking Agent Flavor (Robertet) | 0.4139 | 0.6230 | 0.2129 | 1.0647 |
| Succulence (IFF) | 0.2579 | 0.3882 | 0.1327 | 0.6634 |
| Polysorbate 80 NF | 0.4504 | 0.6779 | 0.2317 | 1.1586 |
| Atmos 300 | 0.4504 | 0.6779 | 0.2317 | 1.1586 |
| Glycerine | 1.9305 | 2.9057 | 0.9932 | 4.9658 |
| Mannitol USP | 2.5740 | 3.8742 | 1.3242 | 6.6211 |
| Sucralose | 1.8000 | 2.7093 | 0.9260 | 4.6301 |
| | | | | |
| | | | | |
| Total | 66.4387 | 100.0000 | 100.0000 | 500.0000 |
| *Assuming that all water is evaporated | | | | |

### EXAMPLE 9

The ingredients listed in Table 9 were combined to provide a consumable film of the present invention in accordance with the following procedure:
A) Dextromethorphan HBr was mixed and dissolved in 90% water to yield an aqueous phase. Pectin dispersed in glycerine was added very slowly to the aqueous phase and mixed at a high mixing rate. The aqueous phase was allowed to cool to about 50ºC and q.s. with water to replace loss due to evaporation. The dye was then added to the aqueous phase and mixed thoroughly.
B) The film-forming ingredients, xanthan gum, locust bean gum, carrageenan and pullulan were mixed together in a separate container to form a film forming mixture.
C) The film forming mixture was slowly added to the aqueous phase of A), followed by overnight mixing at a low mixing rate to provide a hydrated polymer gel.
D) The flavorants, menthol, and surfactants were combined and mixed in a separate container until dissolved to yield an organic phase.
E) Mannitol and sucralose were mixed together in the remaining 10% water in a separate container. Succulence was then added to the resulting mixture and dissolved.
F) The mixtures of steps D) and E) were added to the hydrated polymer gel and mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel).

**Table 9**

| Material | mg/dose* | %w/w* Dry Film | %w/w Actual Batch | g/batch |
|---|---|---|---|---|
| Dextromethorphan (Spectrum) | 10.9900 | 18.3460 | 5.5038 | 27.5189 |
| Pectin USP | 0.5250 | 0.8764 | 0.2629 | 1.3146 |
| Carmine | 0.1900 | 0.3172 | 0.0952 | 0.4758 |
| Xanthan Gum | 0.1154 | 0.1926 | 0.0578 | 0.2888 |
| Locust Bean Gum | 0.1352 | 0.2256 | 0.0677 | 0.3384 |
| Carrageenan | 0.5792 | 0.9668 | 0.2900 | 1.4502 |
| Pullulan | 30.8879 | 51.5621 | 15.4686 | 77.3431 |
| Purified water | - | - | 70 | 350.0000 |
| Menthol | 2.5740 | 4.2969 | 1.2891 | 6.4453 |
| Peppermint Flavor | 0.8000 | 1.3355 | 0.4006 | 2.0032 |
| Cherry Flavor (Givudan) | 0.8000 | 1.3355 | 0.4006 | 2.0032 |
| Sour Cherry (IFF) | 2.2350 | 3.7310 | 1.1193 | 5.5964 |
| Warm Sensation (Mane) | 0.8000 | 1.3355 | 0.4006 | 2.0032 |
| Artificial Masking Agent Flavor (Robertet) | 0.8000 | 1.3355 | 0.4006 | 2.0032 |
| Succulence (IFF) | 0.2579 | 0.4305 | 0.1292 | 0.6458 |
| Polysorbate 80 NF | 0.4504 | 0.7519 | 0.2256 | 1.1278 |
| Atmos 300 | 0.4504 | 0.7519 | 0.2256 | 1.1278 |
| Glycerine | 2.0400 | 3.4054 | 1.0216 | 5.1082 |
| Sucralose | 2.7000 | 4.5072 | 1.3522 | 6.7608 |
| Mannitol USP | 2.5740 | 4.2969 | 1.2891 | 6.4453 |
| | | | | |
| | | | | |
| Total | 59.9042 | 100.0000 | 100.0000 | 500.0000 |
| *Assuming that all water is evaporated | | | | |

### EXAMPLE 10

The ingredients listed in Table 10 were combined to provide a consumable film of the present invention in accordance with the procedure of Example 7.

**Table 10**

| Material | mg/dose* | %w/w* Dry Film | %w/w Actual Batch | g/batch |
|---|---|---|---|---|
| Dextromethorphan (milled) | 10.9900 | 26.6157 | 9.2695 | 18.5390 |
| Amberlite IRP69 | 2.4000 | 5.8123 | 2.0243 | 4.04486 |
| Pectin USP | 0.2698 | 0.6534 | 0.2276 | 0.4551 |
| Carmine | 0.1464 | 0.3546 | 0.1235 | 0.2470 |
| Xanthan Gum | 0.0594 | 0.1439 | 0.0501 | 0.1002 |
| Locust Bean Gum | 0.0694 | 0.1681 | 0.0585 | 0.1171 |
| Carrageenan | 0.2975 | 0.7205 | 0.2509 | 0.5019 |
| Pullulan | 15.8694 | 38.4327 | 13.3850 | 26.7701 |
| Purified water | - | - | 65.1728 | 130.3456 |
| Menthol | 2.5740 | 6.2337 | 2.1710 | 4.3421 |
| Peppermint Flavor | 0.1987 | 0.4812 | 0.1676 | 0.3352 |
| Cherry Flavor (Givudan) | 0.1987 | 0.4812 | 0.1676 | 0.3352 |
| Sour Cherry (IFF) | 1.7225 | 4.1716 | 1.4528 | 2.9057 |
| Warm Sensation (Mane) | 0.4252 | 1.0298 | 0.3586 | 0.7173 |
| Artificial Masking Agent Flavor (Robertet) | 0.3190 | 0.7726 | 0.2691 | 0.5381 |
| Succulence (IFF) | 0.1987 | 0.4812 | 0.1676 | 0.3352 |
| Polysorbate 80 NF | 0.3470 | 0.8404 | 0.2927 | 0.5854 |
| Atmos 300 | 0.3470 | 0.8404 | 0.2927 | 0.5854 |
| Glycerine | 1.4877 | 3.6029 | 1.2548 | 2.5096 |
| Mannitol USP | 1.9837 | 4.8041 | 1.6732 | 3.3463 |
| Sucralose | 1.3873 | 3.3598 | 1.1701 | 2.3402 |
| | | | | |
| | | | | |
| Total | 41.2914 | 100.0000 | 100.0000 | 200.0000 |
| *Assuming that all water is evaporated | | | | |

### EXAMPLE 11

The ingredients listed in Table 11 were combined to provide a consumable film of the present invention in accordance with the following procedure:
A) Dextromethorphan HBr was mixed and dissolved in 90% water to yield an aqueous phase at 75ºC. The Amberlite resin was added to the aqueous phase and mixed for about 4 hours at 70ºC to 80ºC. The aqueous phase was allowed to cool to 50ºC and q.s. with water to replace loss due to evaporation.
B) Pectin was dispersed in glycerine and the resulting mixture was added very slowly to the aqueous phase and mixed at a high mixing rate.
C) The film-forming ingredients, xanthan gum, locust bean gum, carrageenan and pullulan were mixed together in a separate container to form a film forming mixture. The film forming mixture was slowly added to the aqueous phase while mixing rapidly. The resulting mixture was mixed overnight at low speed.
D) In a separate container, sodium chloride, mannitol and sucralose was added to the remaining 10% water. Succulence was then added to the mixture to yield a slurry. The slurry was added to the resulting mixture of step C).
E) The flavorants, menthol, and surfactants were combined and mixed in a separate container until dissolved.
F) The mixtures of steps D) and E) were mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel).

**Table 11**

| Material | mg/dose* | %w/w* Dry Film | %w/w Actual Batch | g/batch |
|---|---|---|---|---|
| Dextromethorphan HBr | 15.0000 | 22.4137 | 7.1724 | 35.8619 |
| Sodium Bicarbonate | 4.0000 | 5.9770 | 1.9126 | 9.5632 |
| Amberlite IRP69 | 8.0000 | 11.9540 | 3.8253 | 19.1264 |
| Pectin USP | 0.3500 | 0.5230 | 0.1674 | 0.8368 |
| Yellow #6 | 0.0200 | 0.0299 | 0.0096 | 0.0478 |
| Xanthan Gum | 0.0500 | 0.0747 | 0.0239 | 0.1195 |
| Locust Bean Gum | 0.1000 | 0.1494 | 0.0478 | 0.2391 |
| Carrageenan | 0.5000 | 0.7471 | 0.2391 | 1.1954 |
| Pullulan | 23.3333 | 34.8657 | 11.1570 | 55.7852 |
| Purified water | - | - | 68.0000 | 340.0000 |
| Menthol | 2.5700 | 3.8402 | 1.2289 | 6.1443 |
| Tangerine Oil | 0.5000 | 0.7471 | 0.2391 | 1.1954 |
| Natural and Artificial Orange | 0.3000 | 0.4483 | 0.1434 | 0.7172 |
| Artificial Lemon Oil | 0.3000 | 0.4483 | 0.1434 | 0.7172 |
| Warm Sensation (Mane) | 0.4000 | 0.5977 | 0.1913 | 0.9563 |
| Artificial Masking Agent Flavor (Robertet) | 0.50000 | 0.7471 | 0.2391 | 1.1954 |
| Succulence (IFF) | 0.3000 | 0.4483 | 0.1434 | 0.7172 |
| Polysorbate 80 NF | 0.6000 | 0.8965 | 0.2869 | 1.4345 |
| Atmos 300 | 0.6000 | 0.8965 | 0.2869 | 1.4345 |
| Glycerine | 2.0000 | 2.9885 | 0.9563 | 4.7816 |
| Sucralose | 2.7000 | 4.0345 | 1.2910 | 6.4552 |
| Mannitol USP | 3.8000 | 5.6781 | 1.8170 | 9.0850 |
| Sodium Chloride | 1.0000 | 1.4942 | 0.4782 | 2.3908 |
| | | | | |
| | | | | |
| Total | 66.9233 | 100.0000 | 100.0000 | 500.0000 |
| *Assuming that all water is evaporated | | | | |

### EXAMPLE 12

The ingredients listed in Table 12 were combined to provide a consumable film of the present invention in accordance with the following procedure:
A) Dextromethorphan HBr was mixed and dissolved in 90% water at 75ºC to yield an aqueous phase. Sodium bicarbonate was added and mixed for about 1 hour. Amberlite IRP69 was added to the aqueous phase and stirred for about 2 hours at about 70ºC to 80ºC. The resulting mixture was allowed to cool to 50ºC and q.s. with water for losses due to evaporation. The dye was then added to the aqueous phase and mixed thoroughly.
B) The film-forming ingredients, xanthan gum, locust bean gum, carrageenan and pullulan were added slowly and rapidly mixed together in a separate container to form a film forming mixture. The mixture was mixed overnight at a low speed. Pectin dispersed in glycerine was added very slowly to the a film forming mixture and mixed at a high mixing rate.
C) The film forming mixture was slowly added to the aqueous phase of A), followed by overnight mixing at a low mixing rate to provide a hydrated polymer gel.
D) In another container the remaining 10% water was added to dissolve mannitol and sucralose. Succulence was then added and mixed to dissolve. The resulting mixture was added to the hydrated polymer gel.
E) The flavorants, menthol, and surfactants were combined and mixed in a separate container until dissolved to yield an organic phase.
F) The mixtures of steps D) and E) were added together and mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel).

**Table 12**

| Material | mg/dose* | %w/w* Dry Film | %w/w Actual Batch | g/batch |
|---|---|---|---|---|
| Dextromethorphan HBr | 15.0000 | 27.3219 | 9.6903 | 484.5135 |
| Amberlite IRP69 | 8.0000 | 14.5717 | 5.1681 | 258.4072 |
| Pectin USP | 0.2698 | 0.4914 | 0.1743 | 8.7148 |
| Sodium bicarbonate anhydrous | 4.0000 | 7.2858 | 2.5841 | 129.2036 |
| Carmine | 0.1464 | 0.2667 | 0.0946 | 4.7289 |
| Xanthan Gum | 0.0594 | 0.1082 | 0.0384 | 1.91187 |
| Locust Bean Gum | 0.0694 | 0.1264 | 0.0448 | 2.2417 |
| Carrageenan | 0.2975 | 0.5419 | 0.1922 | 9.6095 |
| Pullulan | 15.8690 | 28.9047 | 10.2517 | 512.5830 |
| Purified water | - | - | 64.5329 | 3226.6450 |
| Menthol | 2.5740 | 4.6884 | 1.6629 | 83.1425 |
| Peppermint Flavor | 0.1987 | 0.3619 | 0.1284 | 6.4182 |
| Cherry Flavor (Givudan) | 0.1987 | 0.3619 | 0.1284 | 6.4182 |
| Cherry Flavor Blend (IFF) | 1.7225 | 3.1375 | 1.1128 | 55.6383 |
| Warm Sensation (Mane) | 0.4252 | 0.7745 | 0.2747 | 13.7343 |
| Artificial Masking Agent Flavor (Robertet) | 0.3190 | 0.5810 | 0.2061 | 10.3040 |
| Succulence (IFF) | 0.1987 | 0.3619 | 0.1284 | 6.4182 |
| Polysorbate 80 NF | 0.3470 | 0.6320 | 0.2242 | 11.2084 |
| Atmos 300 | 0.3470 | 0.6320 | 0.2242 | 11.2084 |
| Glycerine | 1.4877 | 2.7100 | 0.9611 | 48.0573 |
| Mannitol USP | 1.9837 | 3.6132 | 1.2815 | 64.0753 |
| Sucralose | 1.3873 | 2.5269 | 0.8962 | 44.8110 |
| | | | | |
| | | | | |
| Total | 54.9011 | 100.0000 | 100.0000 | 50000.0000 |
| *Assuming that all water is evaporated | | | | |

### EXAMPLE 13

The ingredients listed in Table 13 were combined to provide a consumable film of the present invention in accordance with the procedure of Example 11, except methyl salicylate, eucalyptol, and thymol were also added to the flavorants, menthol, and surfactants in Step E).

**Table 13**

| Material | mg/dose* | %w/w* Dry Film | %w/w Actual Batch | g/batch |
|---|---|---|---|---|
| Dextromethorphan HBr | 15.0000 | 22.1962 | 7.1028 | 35.5139 |
| Sodium Bicarbonate | 4.0000 | 5.9190 | 1.8941 | 9.4704 |
| Amberlite IRP69 | 8.0000 | 11.8380 | 3.7882 | 18.9408 |
| Pectin USP | 0.3500 | 0.5179 | 0.1657 | 0.8287 |
| Yellow #6 | 0.0200 | 0.0296 | 0.0095 | 0.0474 |
| Xanthan Gum | 0.0500 | 0.0740 | 0.0237 | 0.1184 |
| Locust Bean Gum | 0.1000 | 0.1480 | 0.0474 | 0.2368 |
| Carrageenan | 0.5000 | 0.7399 | 0.2368 | 1.1838 |
| Pullulan | 23.3333 | 34.5274 | 11.0488 | 55.2438 |
| Purified water | - | - | 68.0000 | 340.0000 |
| Thymol | 0.1698 | 0.2513 | 0.0804 | 0.4020 |
| Methyl Salicylate | 0.2430 | 0.3596 | 0.1151 | 0.5753 |
| Eucalyptol | 0.2430 | 0.3596 | 0.1151 | 0.5753 |
| Menthol | 2.5700 | 3.8030 | 1.2169 | 6.0847 |
| Tangerine Oil | 0.5000 | 0.7399 | 0.2368 | 1.1838 |
| Natural and Artificial Orange | 0.3000 | 0.4439 | 0.1421 | 0.7103 |
| Artificial Lemon Oil | 0.3000 | 0.4439 | 0.1421 | 0.7103 |
| Warm Sensation (Mane) | 0.4000 | 0.5919 | 0.1894 | 0.9470 |
| Artificial Masking Agent Flavor (Robertet) | 0.50000 | 0.7399 | 0.2368 | 1.1838 |
| Succulence (IFF) | 0.3000 | 0.4439 | 0.1421 | 0.7103 |
| Polysorbate 80 NF | 0.6000 | 0.8878 | 0.2841 | 1.4206 |
| Atmos 300 | 0.6000 | 0.8878 | 0.2841 | 1.4206 |
| Glycerine | 2.0000 | 2.9595 | 0.9470 | 4.7352 |
| Sucralose | 2.7000 | 3.9953 | 1.2785 | 6.3925 |
| Mannitol USP | 3.8000 | 5.6230 | 1.7994 | 8.9969 |
| Sodium Chloride | 1.0000 | 1.4797 | 0.4735 | 2.3676 |
| Total | 67.5791 | 100.0000 | 100.0000 | 500.0000 |
| *Assuming that all water is evaporated | | | | |

### EXAMPLE 14

The ingredients listed in Table 14 were combined to provide a consumable film of the present invention in accordance with the following procedure:
A) Dextromethorphan HBr was mixed and dissolved in 90% water to yield an aqueous phase at 75ºC. Sodium hydroxide was added to the aqueous phase and thoroughly mixed. The Amberlite resin was then added to the aqueous phase and mixed for about 4 hours at 70ºC to 80ºC. The aqueous phase was allowed to cool to 50ºC and q.s. with water to replace loss due to evaporation.
B) Pectin was added very slowly to the aqueous phase while mixing at a high mixing rate.
C) The film-forming ingredients, xanthan gum, locust bean gum, carrageenan and pullulan were mixed together in a separate container to form a film forming mixture. The film forming mixture was slowly added to the aqueous phase while mixing rapidly. The resulting mixture was mixed overnight at low speed.
D) In a separate container, mannitol and sucralose were added to the remaining 10% water. Succulence was then added to the mixture to yield a slurry. The slurry was added to the resulting mixture of step C).
E) The flavorants, menthol, and surfactants were combined and mixed in a separate container until dissolved.
F) The mixtures of steps D) and E) were mixed uniformly to yield a final polymer gel mixture. The final polymer gel mixture was poured on a mold and cast to form a film of a desired thickness at room temperature. The consumable film was dried under warm air and cut to a desired dimension (dictated by e.g., dosage and mouthfeel).

**Table 14**

| Material | mg/dose* | %w/w* Dry Film | %w/w Actual Batch | g/batch |
|---|---|---|---|---|
| Dextromethorphan HBr | 15.0000 | 23.1042 | 7.3933 | 36.9667 |
| Sodium hydroxide 1 N solution | 5.0000 | 7.7014 | 2.4644 | 12.3222 |
| Amberlite IRP69 | 8.0000 | 12.3222 | 3.9431 | 19.7156 |
| Pectin USP | 0.3500 | 0.5391 | 0.1725 | 0.8626 |
| Yellow #6 | 0.0200 | 0.0308 | 0.0099 | 0.0493 |
| Xanthan Gum | 0.0500 | 0.0770 | 0.0246 | 0.1232 |
| Locust Bean Gum | 0.1000 | 0.1540 | 0.0493 | 0.2464 |
| Carrageenan | 0.5000 | 0.7701 | 0.2464 | 1.2322 |
| Pullulan | 23.3333 | 35.9398 | 11.5007 | 57.5037 |
| Purified water | - | - | 68.0000 | 340.0000 |
| Menthol | 2.5700 | 3.9585 | 1.2667 | 6.3336 |
| Tangerine Oil | 0.5000 | 0.7701 | 0.2464 | 1.2322 |
| Natural and Artificial Orange | 0.3000 | 0.4621 | 0.1479 | 0.7393 |
| Artificial Lemon Oil | 0.3000 | 0.4621 | 0.1479 | 0.7393 |
| Warm Sensation (Mane) | 0.4000 | 0.6161 | 0.1972 | 0.9858 |
| Artificial Masking Agent Flavor (Robertet) | 0.5000 | 0.7701 | 0.2464 | 1.2322 |
| Succulence (IFF) | 0.3000 | 0.4621 | 0.1479 | 0.7393 |
| Polysorbate 80 NF | 0.6000 | 0.9242 | 0.2957 | 1.4787 |
| Atmos 300 | 0.6000 | 0.9242 | 0.2957 | 1.4787 |
| Sucralose | 2.7000 | 4.1588 | 1.3308 | 6.6540 |
| Mannitol USP | 3.8000 | 5.8531 | 1.8730 | 9.3649 |
| | | | | |
| | | | | |
| Total | 64.9233 | 100.0000 | 100.0000 | 500.0000 |
| *Assuming that all water is evaporated | | | | |

### EXAMPLE 15

The ingredients listed in Table 15 were combined to provide a consumable film of the present invention in accordance with the procedure Example 14, except methyl salicylate, eucalyptol, and thymol were also added to the flavorants, menthol, and surfactants in Step E).

**Table 15**

| Material | mg/dose* | %w/w* Dry Film | %w/w Actual Batch | g/batch |
|---|---|---|---|---|
| Dextromethorphan HBr | 15.0000 | 22.7690 | 7.2861 | 36.4304 |
| Sodium hydroxide 1 N solution | 4.0000 | 7.5897 | 2.4287 | 12.1435 |
| Amberlite IRP69 | 8.0000 | 12.1435 | 3.8859 | 19.4295 |
| Pectin USP | 0.3500 | 0.5313 | 0.1700 | 0.8500 |
| Yellow #6 | 0.0200 | 0.0304 | 0.0097 | 0.0486 |
| Xanthan Gum | 0.0500 | 0.0759 | 0.0243 | 0.1214 |
| Locust Bean Gum | 0.1000 | 0.1518 | 0.0486 | 0.2429 |
| Carrageenan | 0.5000 | 0.7590 | 0.2429 | 1.2143 |
| Pullulan | 23.3333 | 35.4184 | 11.3339 | 56.6694 |
| Purified water | - | - | 68.0000 | 340.0000 |
| Thymol | 0.1698 | 0.2577 | 0.0825 | 0.4124 |
| Methyl Salicylate | 0.2430 | 0.3689 | 0.1180 | 0.5902 |
| Eucalyptol | 0.2430 | 0.3689 | 0.1180 | 0.5902 |
| Menthol | 2.8700 | 4.3565 | 1.3941 | 6.9703 |
| Tangerine Oil | 0.5000 | 0.7590 | 0.2429 | 1.2143 |
| Natural and Artificial Orange | 0.3000 | 0.4554 | 0.1457 | 0.7286 |
| Artificial Lemon Oil | 0.3000 | 0.4554 | 0.1457 | 0.7286 |
| Warm Sensation (Mane) | 0.4000 | 0.6072 | 0.1943 | 0.9715 |
| Artificial Masking Agent Flavor (Robertet) | 0.50000 | 0.7590 | 0.2429 | 1.2143 |
| Succulence (IFF) | 0.3000 | 0.4554 | 0.1457 | 0.7286 |
| Polysorbate 80 NF | 0.6000 | 0.9108 | 0.2914 | 1.4572 |
| Atmos 300 | 0.6000 | 0.9108 | 0.2914 | 1.4572 |
| Sucralose | 2.7000 | 4.0984 | 1.3115 | 6.5575 |
| Mannitol USP | 3.8000 | 5.7681 | 1.8458 | 9.2290 |
| Total | 67.5791 | 100.0000 | 100.0000 | 500.0000 |
| *Assuming that all water is evaporated | | | | |

The forgoing discussion discloses and describes merely exemplary embodiments of the present invention. One skilled in the art will readily recognize from such discussion, and from the accompanying claims, that various changes, modifications, and variations can be made therein without departing from the scope of the invention as defined in the following claims.

## Claims

1. A consumable film adapted to adhere to and dissolve in the oral cavity of a consumer comprising at least one water soluble polymer; at least one stabilizing agent selected from the group consisting of xanthan gum, locust bean gum and carrageenan; at least one antitussive agent; and from 0.1 to 2% of a mucosa-coating agent comprising pectin wherein the films are shaped and sized to be placed in the oral cavity.

2. The consumable film of claim 1 wherein the at least one water soluble polymer is selected from the group consisting of pullulan, hydroxypropylmethyl cellulose; hydroxyethyl cellulose, hydroxypropyl cellulose, polyvinyl pyrrolidone, carboxymethyl cellulose, polyvinyl alcohol, sodium alginate, polyethylene glycol, tragacanth gum, guar gum, acacia gum, arabic gum, polyacrylic acid, methylmethacrylate copolymer, carboxyvinyl polymer, amylose, high amylose starch, hydroxypropylated high amylose starch, dextrin, chitin, chitosan, levan, elsinan, collagen, zein, gluten, soy protein isolate, whey protein isolate, casein and combinations thereof.

3. The consumable film of claim 2 wherein said at least one water soluble polymer is pullulan.

4. The consumable film of claim 3 wherein the amount of pullulan is from 0.01% to 99% by weight based on the total weight of the consumable film.

5. The consumable film of claim 1 wherein the antitussive agent is selected from the group consisting of alloclamide, amicibone, benproperine, benzonatate, bibenzonium bromide, bromoform, butamirate, butetamate, caramiphen ethanedisulfonate, caramiphen edisylate, carbetapentane, chlophedianol, clobutinol, cloperastine, codeine, codeine methyl bromide, codeine N-oxide, codeine phosphate, codeine sulfate, cyclexanone, dextromethorphan, dibunate sodium, dihydrocodeine, dihydrocodeinone enol acetate, dimemorfan, dimethoxanate, dropropizine, drotebanol, eprazinone, ethyl dibunate, ethylmorphine, fominoben, guaiapate, hydrocodone, isoaminile, levopropoxyphene, morclofone, narceine, normethadone, noscapine, oxeladin, oxolamine, pholcodine, picoperine, pipazethate, piperidione, prenoxdiazine hydrochloride, racemethorphan, taziprinone hydrochloride, tipepidine, zipeprol and pharmaceutically acceptable salts thereof, and combinations thereof.

6. The consumable film of claim 1 wherein the at least one antitussive agent is dextromethorphan hydrobromide.

7. The consumable film of claim 1 wherein the antitussive agent is present in amounts of from 2,5 mg to 20mg.

8. The consumable film of claim 1 further comprising an antimicrobial effective amount of at least one essential oil selected from the group consisting of carvacrol, thymol, eucalyptol, menthol, methyl salicylate, eugenol, gerianol, verbenone and combinations thereof.

9. The consumable film of claim 8 wherein the antimicrobial effective amount of the at least one essential oil is up to 30% by weight based on the total weight of the consumable film.

10. The consumable film of claim 9 wherein said essential oil comprises menthol.

11. The consumable film of claim 1 in the form of a single layer.

12. The consumable film of claim 1 further comprising at least one additional therapeutic agent.

## Patentansprüche

1. Essbarer Film, der so adaptiert ist, dass er in der Mundhöhle eines Konsumenten haftet und sich darin löst, umfassend mindestens ein wasserlösliches Polymer; mindestens einen Stabilisator, ausgewählt aus der Gruppe bestehend aus Xanthangummi, Johannisbrotgummi und Carrageen; mindestens ein Antitussivum; und 0,1 bis 2% eines schleimhautbeschichtenden Mittels, das Pektin umfasst, wobei die Filme für die Platzierung in die Mundhöhle geformt und dimensioniert sind.

2. Essbarer Film nach Anspruch 1, wobei das mindestens eine wasserlösliche Polymer aus der Gruppe bestehend aus Pullulan, Hydroxypropylmethylcellulose, Hydroxyethylcellulose, Hydroxypropylcellulose, Polyvinylpyrrolidon, Carboxymethylcellulose, Polyvinylalkohol, Natriumalginat, Polyethylenglykol, Tragantgummi, Guargummi, Akaziengummi, Gummi arabicum, Polyacrylsäure, Methylmethacrylat-Copolymer, Carboxyvinylpolymer, Amylose, hochamylosehaltiger Stärke, hydroxypropylierter hochamylosehaltiger Stärke, Dextrin, Chitin, Chitosan, Levan, Elsinan, Collagen, Zein, Gluten, Sojaproteinisolat, Molkeproteinisolat, Casein und Kombinationen davon ausgewählt ist.

3. Essbarer Film nach Anspruch 2, wobei es sich bei dem mindestens einen wasserlöslichen Polymer um Pullulan handelt.

4. Essbarer Film nach Anspruch 3, wobei die Pullulanmenge 0,01 Gew.-% bis 99 Ges.-% in Bezug auf das Gesamtgewicht des essbaren Films ausmacht.

5. Essbarer Film nach Anspruch 1, wobei das Antitussivum aus der Gruppe bestehend aus Alloclamid, Amicibon, Benproperin, Benzonatat, Bibenzoniumbromid, Bromoform, Butamirat, Butetamat, Caramiphen-Ethandisulfonat, Caramiphen-Edisylat, Carbetapentan, Chlophedianol, Clobutinol, Cloperastin, Codein, Codeinmethylbromid, Codein-N-Oxid, Codeinphosphat, Codeinsulfat, Cyclexanon, Dextromethorphan, Natriumdibunat, Dihydrocodein, Dihydrocodeinon-Enolacetat, Dimemorfan, Dimethoxanat, Dropropizin, Drotebanol, Eprazinon, Ethyldibunat, Ethylmorphin, Forminoben, Guaiapat, Hydrocodon, Isoaminil, Levopropoxyphen, Morclofon, Narcein, Normethadon, Noscapin, Oxeladin, Oxolamin, Pholcodin, Picoperin, Pipazethat, Piperidion, Prenoxdiazin-hydrochlorid, Racemethorphan, Taziprinon-hydrochlorid, Tipepidin, Zipeprol und pharmazeutisch unbedenklichen Salzen davon sowie Kombinationen davon ausgewählt ist.

6. Essbarer Film nach Anspruch 1, wobei es sich bei dem mindestens einen Antitussivum um Dextromethorphanhydrobromid handelt.

7. Essbarer Film nach Anspruch 1, wobei das Antitussivum in Mengen von 2,5 mg bis 20 mg vorliegt.

8. Essbarer Film nach Anspruch 1, der weiterhin eine antimikrobiell wirksame Menge von mindestens einem etherischen Öl, ausgewählt aus der Gruppe bestehend aus Carvacrol, Thymol, Eucalyptol, Menthol, Methylsalicylat, Eugenol, Gerianol, Verbenon und Kombinationen davon, umfasst.

9. Essbarer Film nach Anspruch 8, wobei die antimikrobiell wirksame Menge des mindestens einen etherischen Öls bis zu 30 Gew.-% in Bezug auf das Gesamtgewicht des essbaren Films ausmacht.

10. Essbarer Film nach Anspruch 9, wobei das etherische Öl Menthol umfasst.

11. Essbarer Film nach Anspruch 1 in Form einer Einzelschicht.

12. Essbarer Film nach Anspruch 1, der weiterhin mindestens ein zusätzliches Therapeutikum umfasst.

## Revendications

1. Film consommable adapté pour adhérer et se dissoudre dans la cavité orale d'un consommateur, comprenant au moins un polymère hydrosoluble ; au moins un agent stabilisant choisi dans le groupe constitué par la gomme xanthane, la gomme de caroube et un carraghénane ; au moins un agent antitussif ; et de 0,1 à 2% d'un agent de revêtement de muqueuse comprenant de la pectine, où les films sont mis en forme et dimensionnés pour être placés dans la cavité orale.

2. Film consommable selon la revendication 1, dans lequel le au moins un polymère hydrosoluble est choisi dans le groupe constitué par le pullulane, l'hydroxypropyl méthylcellulose, l'hydroxyéthylcellulose, l'hydroxypropylcellulose, la polyvinylpyrrolidone, la carboxyméthylcellulose, l'alcool polyvinylique, l'alginate de sodium, le polyéthylène glycol, la gomme adragante, la gomme guar, la gomme d'acacia, la gomme arabique, l'acide polyacrylique, un copolymère de méthacrylate de méthyle, un polymère de carboxyvinyle, l'amylose, l'amidon riche en amylose, l'amidon hydroxypropylé riche en amylose, les dextrines, la chitine, le chitosane, le lévane, l'elsinane, le collagène, la zéine, le gluten, un isolat de protéines de soja, un isolat de protéines de lactosérum, une caséine, et des combinaisons de ceux-ci.

3. Film consommable selon la revendication 2, dans lequel ledit au moins un polymère hydrosoluble est le pullulane.

4. Film consommable selon la revendication 3, dans lequel la quantité de pullulane va de 0,01% à 99% en poids sur la base du poids total du film consommable.

5. Film consommable selon la revendication 1, dans lequel l'agent antitussif est choisi dans le groupe constitué par l'alloclamide, l'amicibone, la benpropérine, le benzonatate, le bromure de bibenzonium, le bromoforme, le butamirate, le butétamate, l'éthanedisulfonate de caramiphène, l'édisylate de caramiphène, le carbétapentane, le chlophédianol, le clobutinol, la clopérastine, la codéine, le méthylbromure de codéine, le N-oxyde de codéine, le phosphate de codéine, le sulfate de codéine, la cyclohexanone, le dextrométhorphane, le dibunate de sodium, la dihydrocodéine, l'énol acétate de dihydrocodéinone, le dimémorfan, le diméthoxanate, la dropropizine, le drotébanol, l'éprazinone, le dibunate d'éthyle, l'éthylmorphine, le fominobène, le guaiapate, l'hydrocodone, l'isoaminile, le lévopropoxyphène, la morclofone, la narcéine, la norméthadone, la noscapine, l'oxéladine, l'oxolamine, la pholcodine, la picopérine, le pipazéthate, la pipéridione, le chlorhydrate de prénoxdiazine, le racéméthorphan, le chlorhydrate de taziprinone, la tipépidine, le zipéprol, et des sels pharmaceutiquement acceptables de ceux-ci, et des combinaisons de ceux-ci.

6. Film consommable selon la revendication 1, dans lequel le au moins un agent antitussif est le bromhydrate de dextrométhorphane.

7. Film consommable selon la revendication 1, dans lequel l'agent antitussif est présent selon des quantités allant de 2,5 mg à 20 mg.

8. Film consommable selon la revendication 1, comprenant en outre une quantité antimicrobienne efficace d'au moins une huile essentielle choisie dans le groupe constitué par le carvacrol, le thymol, l'eucalyptol, le menthol, le salicylate de méthyle, l'eugénol, le gérianol, la verbénone, et des combinaisons de ceux-ci.

9. Film consommable selon la revendication 8, dans lequel la quantité antimicrobienne efficace de la au moins une huile essentielle constitue jusqu'à 30% en poids sur la base du poids total du film consommable.

10. Film consommable selon la revendication 9, dans lequel ladite huile essentielle comprend du menthol.

11. Film consommable selon la revendication 1, sous la forme d'une couche unique.

12. Film consommable selon la revendication 1, comprenant en outre au moins un agent thérapeutique supplémentaire.
